# EUROPEAN PATENT APPLICATION

(11) **EP 1 995 291 A1**
(43) Date of publication of application: **26.11.2008**
(21) Application number: 07713784.2
(22) Date of filing: 02.02.2007
(51) Int. Cl.: C09K 11/06, C07C 15/38, H01L 51/50

(54) **MATERIAL FOR ORGANIC ELECTROLUMINESCENT DEVICE, METHOD FOR PRODUCING SAME AND ORGANIC ELECTROLUMINESCENT DEVICE**

(30) Priority: 23.02.2006 JP 2006046761
(71) Applicant: Idemitsu Kosan Co., Ltd., Chiyoda-ku Tokyo 100-8321 (JP)
(72) Inventor: IKEDA, Kiyoshi, Sodegaura-shi Chiba 299-0293 (JP); SADO, Takayasu, Sodegaura-shi Chiba 299-0293 (JP); KAWAMURA, Hisayuki, Sodegaura-shi Chiba 299-0293 (JP)
(74) Representative: Gille Hrabal Struck Neidlein Prop Roos
(86) International application number: PCT/JP2007/051790
(87) International publication number: WO 2007/097178

(57) **Abstract**

A material for an organic electroluminescence device which can be a cis-isomer or a trans-isomer of structural isomers, the content of the trans-isomer being more than that of the cis-isomer. The ratio (t/c) of the trans-isomer (t) and the cis-isomer (c) is preferably 2 or more.

## Description

### TECHNICAL FIELD

The invention relates to a material for an organic electroluminescence device, a method for producing the same and an organic electroluminescence device using the same.

### BACKGROUND

An organic electroluminescence (hereinafter "electroluminescence" is abbreviated as "EL") device is an emitting device wherein at least an organic emitting layer is interposed between a pair of electrodes. The organic EL device emits the energy produced by recombination of holes injected from an anode and electrons injected from a cathode as light.
The organic EL device is a self-emission device and has been actively studied in recent years due to its various advantages such as high luminous efficiency, low cost, light weight, and thin-profile.

The organic EL device has a disadvantage that the luminance is decreased with running time, and various improvements for suppressing the luminance deterioration are being investigated.
For example, rubrene, which is a material having condensed aromatic rings as a central skeleton, can be used as a luminous material such as a host or dopant for an organic electroluminescent device. However, it is known that these polycyclic condensed aromatic compounds, particularly compounds wherein 3 or more aromatic rings are condensed, are easily isomerized by light or heat and easily react with oxygen, radicals and the like.

Specifically, it is known that the polycyclic condensed aromatic compounds produce oxides by heating and the like at deposition. In contrast, Patent document 1 discloses a half life of emission and the like of an organic EL device can be improved by producing the organic EL device under the conditions where the oxides are not contained therein as less as possible while measuring content of the oxides by mass spectrum analysis. [Patent Document 1] JP-A-2000-100566

The invention has been made to solve the above problems. An object of the invention is to provide a material for an organic EL device which can improve the emission lifetime of the device.

### DISCLOSURE OF THE INVENTION

The inventors made extensive studies and have found that, a material for an organic EL device can improve emission lifetime of the organic EL device which is a mixture of a trans-isomer and a cis-isomer, which are structural isomers, and contains more the trans-isomer than the cis-isomer.

The invention provides the following material for an organic EL device, and the like.
1. A material for an organic electroluminescence device which can be a cis-isomer or a trans-isomer of structural isomers,
   the content of the trans-isomer being more than that of the cis-isomer.
2. The material for an organic electroluminescence device according to 1 wherein the ratio (t/c) of the trans-isomer (t) and the cis-isomer (c) is 2 or more.
3. The material for an organic electroluminescence device according to 1 which is represented by the following formula (I) : 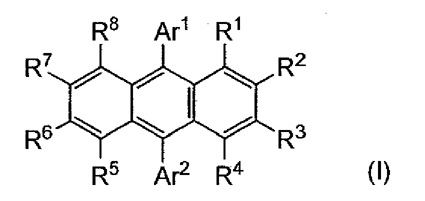 wherein Ar¹ and Ar² are a substituted or unsubstituted aryl group having 6 to 40 carbon atoms that form a ring (ring carbon atoms), or a substituted or unsubstituted heteroaryl group having 5 to 40 atoms that form a ring(ring atoms);
   R¹ to R⁸ are a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
   R¹ to R⁸ may be the same or different, and adjacent groups thereof may form a saturated or unsaturated ring structure; and
   Ar¹ and Ar² have a relation of forming structural isomers.
4. The material for an organic electroluminescence device according to 3 wherein Ar¹ and Ar² of the formula (I) are an aryl group represented by the following formula (II): 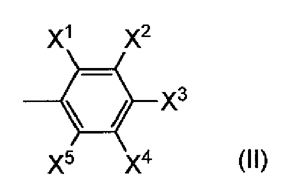 wherein X¹ is a group forming a structural isomer, and is an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
   X² to X⁵ are each independently a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group; and
   X¹ to X⁵ may be the same or different, and adjacent groups thereof may form a saturated or unsaturated ring structure.
5. An organic electroluminescence device comprising:
   a pair of electrodes, and
   one or a plurality of organic layer(s) comprising an organic emitting layer, interposed between the pair of electrodes;
   the organic layer(s) containing the material for an organic device of any one of 1 to 4.
6. The organic electroluminescence device according to 5 wherein the emission zone of the organic layer(s) contain the material for an organic electroluminescence device.
7. The organic electroluminescence device according to 5 wherein the emitting layer contains the material for an organic electroluminescence device.
8. The organic electroluminescence device according to 7 wherein the emitting layer contains the material for an organic electroluminescence device as a main material.
9. A method for producing a material for an organic electroluminescence device comprising:
   heating a mixture of a cis-isomer and trans-isomer of the material which are structural isomers to cause the ratio (t/c) of the trans-isomer (t) and the cis-isomer (c) to be 2 or more.

According to the invention, a structural isomer material suitable for constituent materials of an organic EL device is provided.
According to the invention, a method for producing the material for an organic EL device is provided.

### BRIEF DESCRIPTION OF DRAWINGS

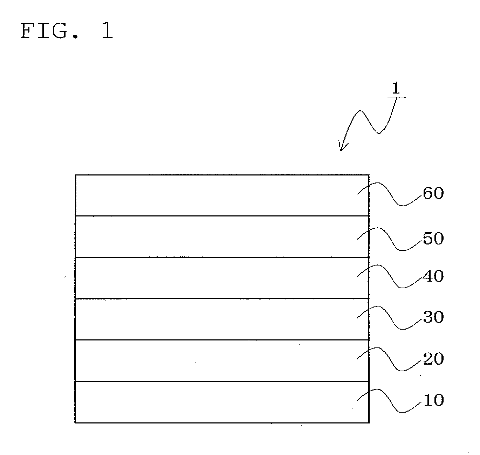

FIG. 1 is a schematic cross-sectional view showing an embodiment of an organic EL device according to the invention.

### BEST MODE FOR CARRYING OUT THE INVENTION

Firstly, a material for an organic EL device of the invention will be described.
The material for an organic EL device of the invention is a material which can be a trans-isomer and a cis-isomer, and the characteristic features of the invention is that the content of the trans-isomer is more than that of the cis-isomer. This material may be a mixture of the trans-isomer and the cis-isomer, or consist of the trans-isomer. The organic EL material which contains the trans-isomer more than the cis-isomer can improve emission half lifetime and emission efficiency.
In the specification, "structural isomers" means molecules which have the same molecular composition and can exist as different molecules from each other due to a steric hinderance existing in their molecular structures. The molecules, which are structural isomers, have the same molecular weight and structural formula as each other, but the molecules often have different properties from each other due to different steric configurations. It is known that these molecules exist as different kinds from each other under general conditions for the use of devices, e.g., under atmospheric pressure and at room temperature, but the molecules change their structure to more a stable structure via an excitation state by light or heating, or under presence of catalysts.

The structural isomers include a cis-isomer and a trans-isomer. In organic chemistry, when a ring of a carbocyclic compound can have an almost plane structure, stereoisomerism occurs by differences in relative position of substituents of this ring to the ring plane. The structural isomer which has substituents positioned on the same side relative to the ring plane is a cis-isomer, and the structural isomer which has substituents positioned on the opposite sides relative to the ring plane is a trans-isomer. That is to say, the cis-isomer means a molecular structure wherein sterically-hindered groups which can produce structural isomers are present in the same direction. In contrast, the trans-isomer means a molecular structure wherein such sterically-hindered groups are present in different directions from each other.

For example, in the case of an anthracene derivative having two phenyl groups which are substituted at 9 position and 10 position with sterically-bulky substituents X^{d}, two structures shown by the following formulas can equivalently exist under the usual use conditions. Among these, the isomer having the structure of (a) is the cis-isomer, and the isomer having the structure of (b) is the trans-isomer:

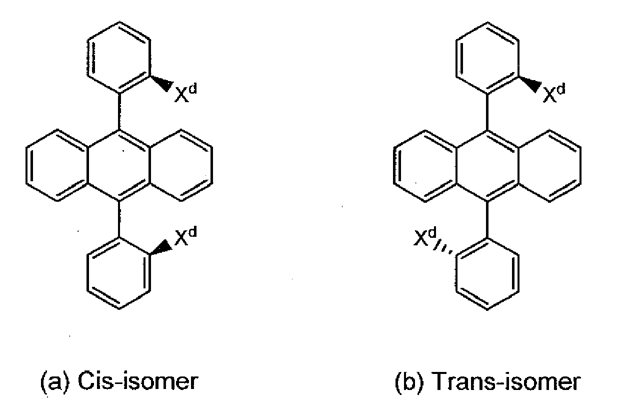

In the material for an organic EL device of the invention, the content of the trans-isomer is more than that of the cis-isomer. That is to say, the ratio (t/c) between the trans-isomer (t) and the cis-isomer (c) is more than 1. The ratio (t/c) is preferably 2 or more, more preferably 5 or more. There is no upper limit of the ratio, and it is further preferable that the material of the invention consist of only the trans-isomer.
The ratio (t/c) means ratio of surface percentages in an HPLC measurement.

The ratio of trans-isomer and cis-isomer can be measured by various methods. For example, the ratio t/c can be determined by focusing attention to a specific hydrogen atom in H-NMR and comparing the peak strengths. As another method, the ratio t/c can be determined by comparing surface percentages of the trans-isomer and the cis-isomer obtained by high-performance liquid chromatography (HPLC). In the specification, the ratio t/c is measured by the latter high-performance liquid chromatography (HPLC).

Specific examples of the material for an organic EL device of the invention include the compounds represented by the following formula (I):

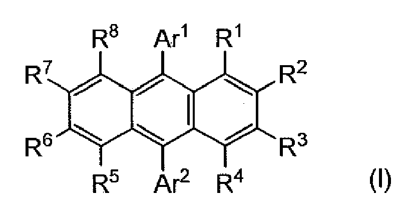

In the formula (I), Ar¹ and Ar² are a substituted or unsubstituted aryl group having 6 to 40 ring carbon atoms, or a substituted or unsubstituted heteroaryl group having 5 to 40 ring atoms;
R¹ to R⁸ are a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
R¹ to R⁸ may be the same or different, and adjacent groups thereof may form a saturated or unsaturated ring structure; and
Ar¹ and Ar² have a relation of forming structural isomers.

In the formula (I), Ar¹ and Ar² are preferably an aryl group represented by the following formula (II):

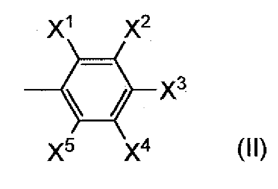

wherein X¹ is a group forming a structural isomer, and is an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
X² to X⁵ are each independently a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group; and
X¹ to X⁵ may be the same or different, and adjacent groups thereof may form a saturated or unsaturated ring structure.

In the formulas (I) and (II), as examples of the alkyl group represented by R¹ to R⁸ and X¹ to X⁵, methyl, ethyl, n-propyl, i-propyl, n-butyl, s-butyl, t-butyl, n-pentyl, cyclopentyl, n-hexyl, cyclohexyl, and adamantyl groups can be given. Of these, methyl, ethyl, n-propyl, i-propyl, and t-butyl groups are preferable.

In the formulas (I) and (II), as examples of the alkoxy group represented by R¹ to R⁸ and X¹ to X⁵, methoxy, ethoxy, i-propyloxy, n-butoxy, s-butoxy, t-butoxy, n-pentoxy, cyclopentoxy, n-hexyloxy, and cyclohexyloxy groups can be given. Of these, a methoxy group is preferable.

In the formulas (I) and (II), as examples of the aryl group represented by R¹ to R⁸ and X¹ to X⁵, phenyl, naphthyl, phenanthryl, anthracenyl, pyrenyl, crycenyl, fluorenyl, and fluoranthenyl groups can be given. Of these, a phenyl group is preferable.

In the formulas (I) and (II), as examples of the heteroaryl gorup represented by R¹ to R⁸ and X¹ to X⁵, pyrrolyl, furanyl, thiophenyl, imidazolyl, benzofuranyl, benzothiophenyl, carbazolyl, pyridinyl, and quinoxanyl groups can be given. Of these, thiophenyl and pyridinyl groups are preferable.

In the formulas (I) and (II), as the halogen atom represented by R¹ to R⁸ and X¹ to X⁵, fluorine and chlorine are preferable.
In the formulas (I) and (II), as examples of the saturated or unsaturated ring structure formed by adjacent groups of R¹ to R⁸ and X¹ to X⁵, benzene and cyclohexane rings can be given.
Specific examples of the compound represented by formula (I) are shown below.

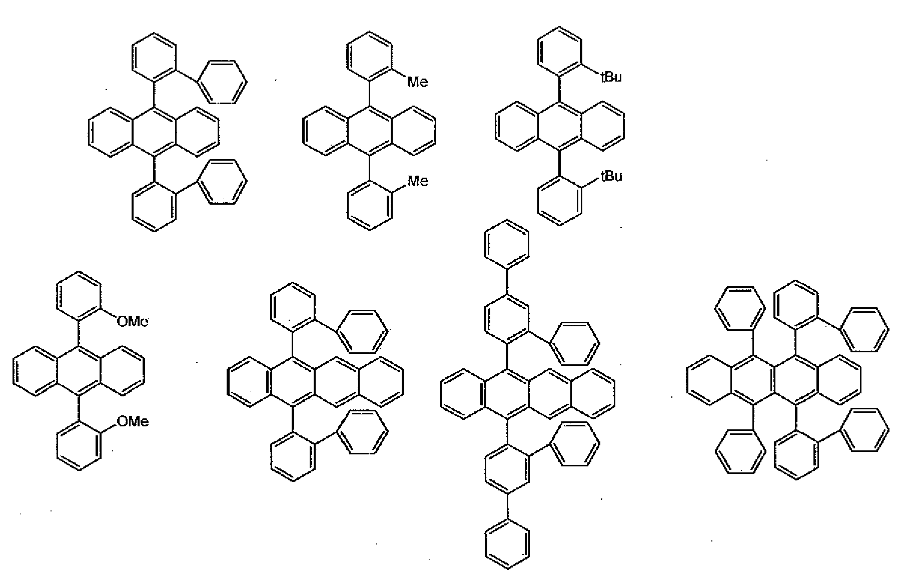

wherein Me is a methyl group and tBu is a tertiary butyl group.

The material for an organic EL device of the invention is obtainable by appropriately adjusting synthesis conditions of compounds having structural isomers. The synthesized compounds are generally obtained as a mixture of the cis-isomer and the trans-isomer.
Since the structural isomers do not undergo structural isomerism changes under general use conditions, the ratio (t/c) of the trans-isomer and the cis-isomer does not change. However, the structural isomers may be isomerized via the excitation state due to irradiation of light, heating or existence of catalysts. The inventors made extensive studies and found that it is effective to heat the synthesized compound, which has structural isomers, for increasing the ratio of the trans-isomer thereof. The materials for an organic EL device of the invention do not undergo structural isomerism changes under general preservation states, which is at room temperature under a light blocking condition.

In the material for an organic EL device, which is a compound having structural isomers, the heating temperature for increasing the trans-isomer is preferably 200 to 400°C, more preferably 250 to 350°C. Although it needs to appropriately adjust the heating time depending on the compound, the time is generally 1 to 50 hours and preferably 1 to 10 hours.
Such heat treatments enable the material for an organic EL device to have the ratio (t/c) of the trans-isomer and the cis-isomer of 2 or more.

From the viewpoint of preventing oxide generation, the heat treatments are preferably conducted in an inert gas atmosphere such as dry nitrogen and argon under atmospheric pressure or a pressure pressurized to more than atmospheric pressure. In contrast, heat treatments and sublimation purification may be conducted under a vacuum of 10⁻² Pa to 10⁻⁶ Pa after replacement to an inert gas atmosphere.
Since oxidizing reaction may be promoted by light, particularly UV irradiation (Document example: Bull. Chemn. Soc. Jpn 61 (1988) p.1057 to 1062), the heat treatments are preferably conducted while blocking the material from exposure to light.

Next, the organic EL device of the invention will be described below.
The organic EL device of the invention comprises a pair of electrodes, and one or a plurality of organic layer(s) comprising an organic emitting layer, interposed between the pair of electrodes.

FIG. 1 is a systematic cross-sectional view showing one embodiment of the organic EL device of the invention.
In the organic EL device 1, an anode 10, a hole-injecting layer 20, a hole-transporting layer 30, an emitting layer 40, an electron-transporting layer 50, and a cathode 60 are stacked on a substrate (not shown) in this order. In this device, the organic thin layer has a stacked structure of the hole-inj ecting layer 20, the hole-transporting layer 30, the emitting layer 40, and the electron-transporting layer 50.

In the organic EL device of the invention, at least one of the organic layers contains the material for an organic EL device of the invention. This leads to an improved luminous efficiency and a prolonged lifetime of the organic EL device.
In the organic layer containing the material for an organic EL device of the invention, the content of the material is adjusted considering the function of the organic layer and is preferably ranged from 1 to 100 mol%, and particularly preferably ranged from 50 to 100 mol%.
The material for an organic EL device preferably exists in an emission zone and is particularly preferably used in an emitting layer.
The emission zone means a region which actually emits light via an excitation state.

The emitting layer preferably contains the above -mentioned material for an organic EL device as a main material. This leads to well balanced recombination of electrons and holes and stable emission.
The "containing as a main material" means that the content of the material for an organic EL device contained in whole the emitting layer is 50 mol% or more and preferably 80 mol% or more.

As mentioned above, it suffices that at least one of the organic layers of the organic EL device of the invention contains the material for an organic EL device of the invention. Therefore, the structure of the device of the invention is not limited to the above embodiment 1. For example, the device may have structures (1) to (15) shown below.
(1) Anode/emitting layer/cathode
(2) Anode/hole-transporting layer/emitting layer/cathode
(3) Anode/emitting layer/hole-transporting layer/cathode
(4) Anode/hole-transporting layer/emitting layer/electron-transporting layer/cathode
(5) Anode/hole-transporting layer/emitting layer/adhesion-improving layer/cathode
(6) Anode/hole-injecting layer/hole-transporting layer/emitting layer/electron-transporting layer/cathode (FIG. 1)
(7) Anode/hole-transporting layer/emitting layer/electron-transporting layer/electron-injecting layer/cathode
(8) Anode/hole-injecting layer/hole-transporting layer/emitting layer/electron-transporting layer/electron-injecting layer/cathode
(9) Anode/insulating layer/hole-transporting layer/emitting layer/electron-transporting layer/cathode
(10) Anode/hole-transporting layer/emitting layer/electron-transporting layer/insulating layer/cathode
(11) Anode/inorganic semiconductor layer/insulating layer/hole-transporting layer/emitting layer/insulating layer/cathode
(12) Anode/insulating layer/hole-transporting layer/emitting layer/electron-transporting layer/insulating layer/cathode
(13) Anode/hole-injecting layer/hole-transporting layer/emitting layer/electron-transporting layer/insulating layer/cathode
(14) Anode/insulating layer/hole-injecting layer/hole-transporting layer/emitting layer/electron-transporting layer/electron-injecting layer/cathode
(15) Anode/insulating layer/hole-injecting layer/hole-transporting layer/emitting layer/electron-transporting layer/electron-injecting layer/insulating layer/cathode

Among these, usually, the structures (4), (6), (7), (8), (12), (13) and (15) are preferably used.
Each member constituting the organic EL device of the invention will be described below.

### (Transparent substrate)

The organic EL device is formed on a transparent substrate. The transparent substrate is a substrate for supporting the organic EL device, and is preferably a flat and smooth substrate having a 400-to-700-nm-visible-light transmittance of 50% or more.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfide, and polysulfone.
Transparency is not required when the supporting substrate is positioned in the direction opposite to the light-outcoupling direction.

### (Anode)

The anode of the organic EL device plays a role for injecting holes into its hole-transporting layer or emitting layer. When transparency is required for the anode, indium tin oxide alloy (ITO), tin oxide (NESA), zinc tin oxide alloy (IZO), gold, silver, platinum, copper, and the like may be used as the material for the anode. When the anode is a reflective electrode which does not require transparency, a metal such as aluminum, molybdenum, chromium, and nickel or alloys thereof may also be used. Although these materials may be used individually, alloys thereof or materials wherein another element is added to the materials can be selected for use.
The anode can be formed by forming these electrode materials into a thin film by vapor deposition, sputtering or the like.
In the case where light is emitted from the emitting layer through the anode, the light transmittance of the anode is preferably more than 10%. The sheet resistance of the anode is preferably several hundred Ω/□ or less. The film thickness of the anode, which varies depending upon the material thereof, is usually from 10 nm to 1 µm, preferably from 10 to 200 nm.

### (Emitting layer)

The emitting layer of the organic EL device has the following functions in combination.
(1) Injection function: function of allowing injection of holes from the anode or hole-injecting/transporting layer and injection of electrons from the cathode or electron-injecting/transporting layer upon application of an electric field
(2) Transporting function: function of moving injected carriers (electrons and holes) with the force of an electric field
(3) Emitting function: function of allowing electrons and holes to recombine therein to emit light

Note that electrons and holes may be injected into the emitting layer with different degrees, or the transportation capabilities indicated by the mobility of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film.
The term "molecular deposition film" refers to a thin film formed by depositing a vapor-phase material compound or a film formed by solidifying a solution-state or liquid-phase material compound. The molecular deposition film is generally distinguished from a thin film (molecular accumulation film) formed using the LB method by a difference in aggregation structure or higher order structure, or a difference in function due to the difference in structure.
The emitting layer may also be formed by dissolving a binder such as a resin and a material compound in a solvent to obtain a solution, and forming a thin film from the solution by spin coating or the like, as disclosed in JP-A-57-51781.

As materials for the emitting layer, the above-mentioned material for an organic EL device of the invention is preferable, but the materials for the emitting layer are not limited to this material. As specific examples thereof, a material represented by the formula (III) can be used as an emitting material: 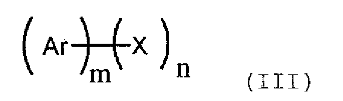 wherein Ar is an aromatic ring with 6 to 50 ring carbon atoms or an aromatic heteroring having 5 to 50 ring atoms, X is a substituent, m is an integer of 1 to 5, and n is an integer of 0 to 6.

As specific examples of the aromatic ring and aromatic heteroring shown by Ar, a phenyl ring, a naphthyl ring, an anthracene ring, a biphenylene ring, an azulene ring, an acenaphthylene ring, a fluorene ring, a phenanthrene ring, a fluoranthene ring, an acephenanthrylene ring, a triphenylene ring, a pyrene ring, a chrysene ring, a benzanthracene ring, a naphthacene ring, a picene ring, a perylene ring, a pentaphene ring, a pentacene ring, a tetraphenylene ring, a hexaphene ring, a hexacene ring, a rubicene ring, a coronene ring, a trinaphthylene ring, a pyrrole ring, an indole ring, a carbazole ring, an imidazole ring, a benzimidazole ring, an oxadiazole ring, a triazole ring, a pyridine ring, a quinoxaline ring, a quinoline ring, a pyrimidine ring, a triazine ring, a thiophene ring, a benzothiophene ring, a thianthrene ring, a furan ring, a benzofuran ring, a pyrazole ring, a pyrazine ring, a pyridazine ring, an indolizine ring, a quinazoline ring, a phenanthroline ring, a silole ring, a benzosilole ring, and the like can be given.

Ar is preferably a phenyl ring, a naphthyl ring, an anthracene ring, an acenaphthylene ring, a fluorene ring, a phenanthrene ring, a fluoranthene ring, a triphenylene ring, a pyrene ring, a chrysene ring, a benzanthracene ring, or a perylene ring.

Specific examples of the substituent represented by X include a substituted or unsubstituted aromatic group having 6 to 50 ring carbon atoms, a substituted or unsubstituted aromatic heterocyclic group having 5 to 50 ring atoms, a substituted or unsubstituted alkyl group having 1 to 50 carbon atoms, a substituted or unsubstituted alkoxy group having 1 to 50 carbon atoms, a substituted or unsubstituted aralkyl group having 1 to 50 carbon atoms, a substituted or unsubstituted aryloxy group having 5 to 50 ring atoms, a substituted or unsubstituted arylthio group having 5 to 50 ring atoms, a substituted or unsubstituted carboxyl group having 1 to 50 carbon atoms, a substituted or unsubstituted styryl group, a halogen group, a cyano group, a nitro group and a hydroxyl group.

As examples of the substituted or unsubstituted aromatic group having 6 to 50 ring carbon atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-fluorenyl group, 9,9-dimethyl-2-fluorenyl group, 3-fluoranthenyl group, and the like can be given.

The substituted or unsubstituted aromatic group having 6 to 50 ring carbon atoms is preferably a phenyl group, 1-naphthyl group, 2-naphthyl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group, p-t-butylphenyl group, 2-fluorenyl group, 9,9-dimethyl-2-fluorenyl group, 3-fluoranthenyl group, or the like.

As examples of the substituted or unsubstituted aromatic heterocyclic group having 5 to 50 ring atoms, a 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 10-phenothiadinyl group, 1-phenoxadinyl group, 2-phenoxadinyl group, 3-phenoxadinyl group, 4-phenoxadinyl group, 10-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, and the like can be given.

Examples of the substituted or unsubstituted alkyl groups having 1 to 50 carbon atoms include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, 1,2,3-trinitropropyl, cyclopropyl, cyclobutyl, cyclopentyl, cyclohexyl, 4-methylcyclohexyl, 1-adamanthyl, 2-adamanthyl, 1-norbornyl, and 2-norbornyl.

The substituted or unsubstituted alkoxy groups having 1 to 50 carbon atoms are groups represented by -OY. Examples of Y include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-Chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, and 1,2,3-trinitropropyl groups.

Examples of the substituted or unsubstitued aralkyl groups having 1 to 50 carbon atoms include benzyl, 1-phenylethyl, 2-phenylethyl, 1-phenylisopropyl, 2-phenylisopropyl, phenyl-t-butyl, α-naphthylmethyl, 1-α-naphthylethyl, 2-α-naphthylethyl, 1-α-naphthylisopropyl, 2-α-naphthylisopropyl, β-naphthylmethyl, 1-β-naphthylethyl, 2-β-naphthylethyl, 1-β-naphthylisopropyl, 2-β-naphthylisopropyl, 1-pyrrolylmethyl, 2-(1-pyrrolyl) ethyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl, and 1-chloro-2-phenylisopropyl groups.

The substituted or unsubstituted aryloxy group having 5 to 50 ring atoms is represented by -OY'. Examples of Y' include a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, and 4-t-butyl-3-indolyl group.

The substituted or unsubstituted arylthio group having 5 to 50 ring atoms is represented by -SY''. Examples of Y" include a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, and 4-t-butyl-3-indolyl group.

The substituted or unsubstituted carboxyl group having 1 to 50 carbon atoms is represented by -COOZ. Examples of Z include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, and 1,2,3-trinitropropyl.

As examples of the substituted or unsubstituted styryl group, 2-phenyl-1-vinyl group, 2,2-diphenyl-1-vinyl group, 1,2,2-triphenyl-1-vinyl group, and the like can be given.

As examples of the halogen group, fluorine, chlorine, bromine, iodine, and the like can be given.

m is preferably 1 to 2. n is preferably 0 to 4. When m≥2, the Ars in the formula (III) may be the same or different. When n≥2, the Xs in the formula (III) may be the same or different.

As the material used in the emitting layer, it is further preferable to use an anthracene derivative represented by the following formula (IV).

A¹-L-A² (IV)

wherein A¹ and A² are independently a substituted or unsubstituted monophenylanthryl group or a substituted or unsubstituted diphenylanthryl group, and may be the same or different; and L is a single bond or a divalent linking group.

In addition to the anthracene derivative described above, an anthracene derivative represented by the formula (V) can be given.

A³-An-A⁴ (V)

wherein An is a substituted or unsubstituted divalent anthracene residue; and A³ and A⁴ are independently a substituted or unsubstituted monovalent condensed aromatic ring or a substituted or unsubstituted non-condensed ring aryl group having 12 or more carbon atoms and may be the same or different.

As preferable anthracene derivatives represented by the formula (IV), anthracene derivatives represented by the formula (IV-a) or the formula (IV-b) can be given, for example. 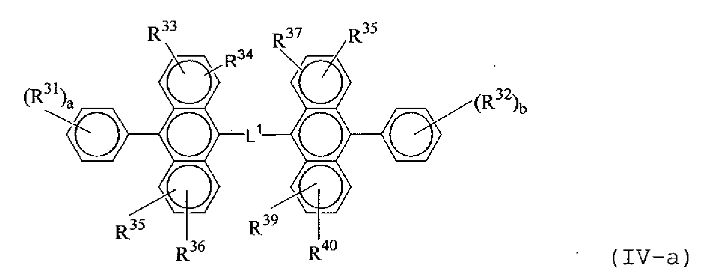 wherein R³¹ to R⁴⁰ are independently a hydrogen atom, an alkyl group, a cycloalkyl group, an aryl group which may be substituted, an alkoxy group, an aryloxy group, an alkylamino group, an arylamino group or a heterocyclic group which may be substituted; a and b are each an integer of 1 to 5; when they are 2 or more, R³¹s or R³²s may be the same or different, or R³¹s or R³²s may be bonded to each other to form a ring; R³³ and R³⁴, R³⁵ and R³⁶, R³⁷ and R³⁸, or R³⁹ and R⁴⁰ may be bonded to each other to form a ring; and L¹ is a single bond, -O-, -S-, -N(R)- (R is an alkyl group or an aryl group which may be substituted), or an arylene group.

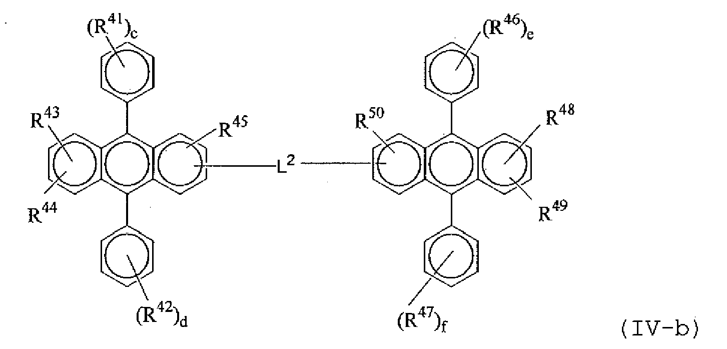

wherein R⁴¹ to R⁵⁰ are independently a hydrogen atom, an alkyl group, a cycloalkyl group, an aryl group which may be substituted, an alkoxy group, an aryloxy group, an alkylamino group, an arylamino group or a heterocyclic group which may be substituted; c, d, e and f are each an integer of 1 to 5; when they are 2 or more, R⁴¹s, R⁴²s, R⁴⁶s or R⁴⁷s may be the same or different, R⁴¹s, R⁴²s, R⁴⁶s or R⁴⁷s may be bonded to each other to form a ring, or R⁴³ and R⁴⁴, or R⁴⁸ and R⁴⁹ may be bonded to each other to form a ring; and L² is a single bond, -O-, -S-, -N(R)- (R is an alkyl group or an aryl group which may be substituted), or an arylene group.
Here, the term "may be substituted" means "a substituted or unsubstituted".

As for R³¹ to R⁵⁰ shown in the above formulas (IV-a) and (IV-b), as the alkyl group, an alkyl group having 1 to 6 carbon atoms, as the cycloalkyl group, a cycloalkyl group having 3 to 6 carbon atoms, as the aryl group, an aryl group having 5 to 18 carbon atoms, as the alkoxy group, an alkoxy group having 1 to 6 carbon atoms, as the aryloxy group, an aryloxy group having 5 to 18 carbon atoms, as the arylamino group, an amino group substituted with an aryl group having 5 to 16 carbon atoms, as the heterocyclic group, triazole, oxadiazole, quinoxaline, furanyl, thienyl or the like can preferably be given.

As the alkyl group and the aryl group shown by R in -N (R) - in L¹ and L², an alkyl group having 1 to 6 carbon atoms and an aryl group having 5 to 18 carbon atoms are preferable.

The emission performance of the emitting layer can be improved by adding a small amount of a fluorescent compound as a dopant therein. As the dopant, known luminescent materials having a long lifetime may be used. It is preferable to use, as the dopant material of the luminescent material, a material represented by the formula (VI): 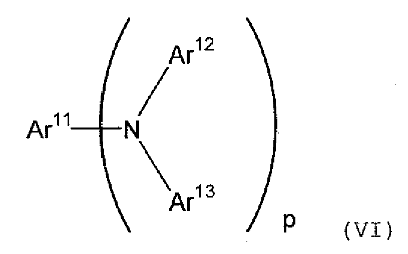 wherein Ar¹¹ to Ar¹³ are independently a substituted or unsubstituted aromatic group with 6 to 50 ring carbon atoms, or a substituted or unsubstituted styryl group.

As examples of the substituted or unsubstituted aromatic group having 6 to 50 ring carbon atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-fluorenyl group, 9,9-dimethyl-2-fluorenyl group, 3-fluoranthenyl group, and the like can be given.

The substituted or unsubstituted aromatic group having 6 to 50 ring carbon atoms is preferably a phenyl group, 1-naphthyl group, 2-naphthyl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group, p-t-butylphenyl group, 2-fluorenyl group, 9,9-dimethyl-2-fluorenyl group, 3-fluoranthenyl group, or the like. As examples of the substituted or unsubstituted styryl group, 2-phenyl-1-vinyl group, 2,2-diphenyl-1-vinyl group, 1,2,2-triphenyl-1-vinyl group, and the like can be given.

p is an integer of 1 to 4. When p≥2, the Ar¹² and Ar¹³ in the formula (VI) may be the same or different.

### (Hole-transporting/injecting layer)

The hole-transporting layer is a layer for helping the injection of holes into the emitting layer so as to transport holes to an emitting region. The hole mobility thereof is large and the ionization energy thereof is usually as small as 5.5 eV or less. Such a hole-transporting layer is preferably made of a material which can transport holes to the emitting layer at a lower electric field intensity and preferably have a hole mobility of at least 10⁻⁴ cm²/V·second when applying an electric field of 10⁴ to 10⁶ V/cm, for example.

Specific examples of hole transporting materials include triazole derivatives (see USP No. 3,112,197 or the like), oxadiazole derivatives (see USP No. 3,189,447 or the like), imidazole derivatives (see JP-B-37-16096 or the like), polyarylalkane derivatives (see USP Nos. 3,615,402, 3,820,989 and 3,542,544, JP-B-45-555 and 51-10983, JP-A-51-93224, 55-17105, 56-4148, 55-108667, 55-156953 and 56-36656, or the like), pyrazoline derivatives and pyrazolone derivatives (see USP Nos. 3,180,729 and 4,278,746, JP-A-55-88064, 55-88065, 49-105537, 55-51086, 56-80051, 56-88141, 57-45545, 54-112637 and 55-74546, or the like), phenylenediamine derivatives (see USP No. 3,615,404, JP-B-51-10105, 46-3712 and 47-25336, JP-A-54-53435, 54-110536 and 54-119925, or the like) , arylamine derivatives (see USP Nos. 3,567,450, 3,180,703, 3,240,597, 3,658,520, 4,232,103, 4,175,961 and 4,012,376, JP-B-49-35702 and 39-27577, JP-A-55-144250, 56-119132 and 56-22437, DE1,110,518, or the like), amino-substituted chalcone derivatives (see USP No. 3,526,501, or the like), oxazole derivatives (ones disclosed in USP No. 3,257,203, or the like), styrylanthracene derivatives (see JP-A-56-46234, or the like), fluorenone derivatives (JP-A-54-110837, or the like), hydrazone derivatives (see USP Nos. 3,717,462, JP-A-54-59143, 55-52063, 55-52064, 55-46760, 55-85495, 57-11350, 57-148749 and 2-311591, or the like), stilbene derivatives (see JP-A-61-210363, 61-228451, 61-14642, 61-72255, 62-47646, 62-36674, 62-10652, 62-30255, 60-93455, 60-94462, 60-174749 and 60-175052, or the like), silazane derivatives (USP No. 4,950,950), polysilanes (JP-A-2-204996), aniline copolymers (JP-A-2-282263), and electroconductive high molecular oligomers (in particular thiophene oligomers) disclosed in JP-A-1-211399.

A compound represented by the following formula is preferable as the hole transporing material: 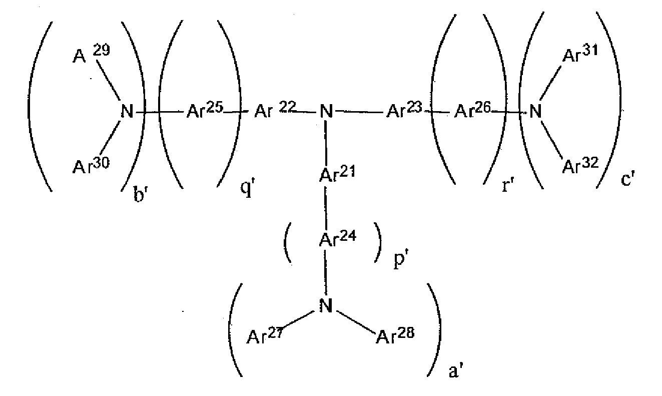 wherein Ar²¹ to Ar²³, Ar²⁴ to Ar²⁶, Ar²⁷ to Ar³² are independently a substituted or unsubstituted aromatic group having 6 to 50 ring carbon atoms or a heteroaromatic group having 5 to 50 ring atoms; a' to c', and p' to r' are independently an integer of 0 to 3; and Ar²⁷ and Ar²⁸, Ar²⁹ and Ar³⁰, and Ar³¹ and Ar³² may be bonded to each other to form a saturated or unsaturated ring.

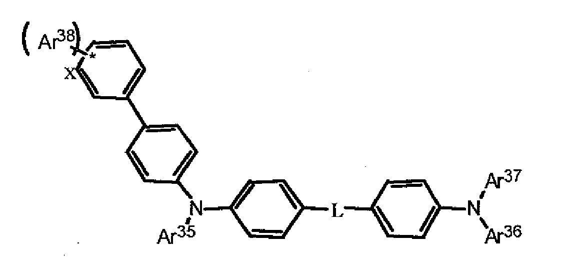

wherein Ar³⁵ to Ar³⁸ are a substituted or unsubstituted aromatic group having 6 to 50 ring carbon atoms or a heteroaromatic group having 5 to 50 ring atoms; L is a linking group, a single bond, a substituted or unsubstituted aromatic group having 6 to 50 ring carbon atoms, or a heteroaromatic group having 5 to 50 ring atoms; x' is an integer of 0 to 5; and Ar³⁶ and Ar³⁷ may be bonded to each other to form a saturated or unsaturated ring.

In addition to the hole-transporting layer, it is preferred that a hole-injecting layer be separately provided to help the injection of holes. As the material for the hole-injecting layer, the organic EL material of the invention may be used singly or in combination with other materials. As other materials, the materials for the hole-transporting layer can be used. The following is preferably used: porphyrin compounds (disclosed in JP-A-63-2956965 and others), aromatic tertiary amine compounds and styrylamine compounds (see USP No. 4,127,412, JP-A-53-27033, 54-58445, 54-149634, 54-64299, 55-79450, 55-144250, 56-119132, 61-295558, 61-98353 and 63-295695, and others) ; particularly preferably, and aromatic tertiary amine compounds.

The following can also be given as examples: 4,4'-bis(N-(1-naphthyl)-N-phenylamino)biphenyl (NPD), which has in the molecule thereof two condensed aromatic rings, disclosed in USP No. 5,061,569, and 4,4',4"-tris(N-(3-methylphenyl)-N-phenylamino) triphenylamine (MTDATA), wherein three triphenylamine units are linked in a star-burst form, disclosed in JP-A-4-308688.

Inorganic compounds such as p-type Si and p-type SiC as well as aromatic dimethylidene type compounds can also be used as the material of the hole-injecting layer.

The hole-injecting layer or hole-transporting layer can be formed from the above-mentioned compounds by a known method such as vacuum deposition, spin coating, casting or LB technique. The film thickness of the hole-injecting layer and hole-transporting layer is not particularly limited, and is usually from 5 nm to 5 µm. The hole-injecting layer or hole-transporting layer may be a single layer made of one, or two or more of the above-mentioned materials, or may be stacked hole-injecting layers or hole-transporting layers made of different compounds, insofar as the compound of the invention is contained.

An organic semiconductor layer is one type of a hole-transporting layer for helping the injection of holes or electrons into an emitting layer, and is preferably a layer having an electric conductivity of 10⁻¹⁰ S/cm or more. As the material of such an organic semiconductor layer, electroconductive oligomers such as thiophene-containing oligomers or arylamine-containing oligomers disclosed in JP-A-8-193191, and electroconductive dendrimers such as arylamine-containing dendrimers may be used.

### (Electron-injecting layer)

The electron-injecting layer, which may be occasionally included in an electron-transporting layer, is a layer for helping the injection of electrons into the emitting layer, and has a large electron mobility. An adhesion-improving layer, which is one type of the electron-injecting layer, is formed of a material which exhibits excellent adhesion to the cathode. The material used in the electron-injecting layer is preferably a metal complex of 8-hydroxyquinoline or a derivative thereof.

As specific examples of a metal complex of an 8-hydroxyquinoline or 8-hydroxyquinoline derivative, metal chelate oxynoid compounds including a chelate of oxine (generally, 8-quinolinol or 8-hydroxyquinoline) can be given. For example, Alq described as the emitting material can be used for the electron-injecting layer.

An electron-transporting compound of the following formula can be given as the oxadiazole derivative. 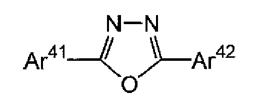 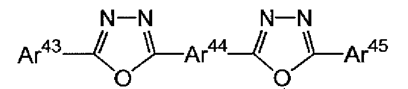 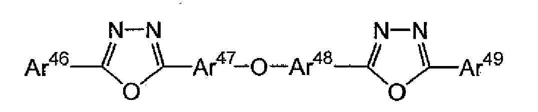 wherein Ar⁴¹, Ar⁴², Ar⁴³, Ar⁴⁵, Ar⁴⁶, and Ar⁴⁹ are independently a substituted or unsubstituted aryl group and may be the same or different. Ar⁴⁴, Ar⁴⁷, and Ar⁴⁸ are independently a substituted or unsubstituted arylene group and may be the same or different.

As examples of the aryl group, a phenyl group, a biphenyl group, an anthranyl group, a perylenyl group, and a pyrenyl group can be given. As examples of the arylene group, a phenylene group, a naphthylene group, a biphenylene group, an anthranylene group, a perylenylene group, a pyrenylene group, and the like can be given. As the substituent, an alkyl group having 1 to 10 carbon atoms, an alkoxy group having 1 to 10 carbon atoms, a cyano group, and the like can be given. The electron transporting compound is preferably one from which a thin film can be formed.

The following compounds can be given as specific examples of the electron transporting compound.

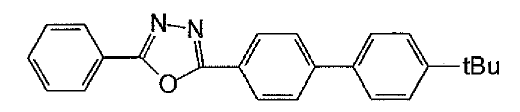

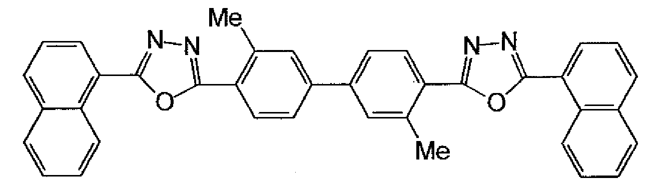

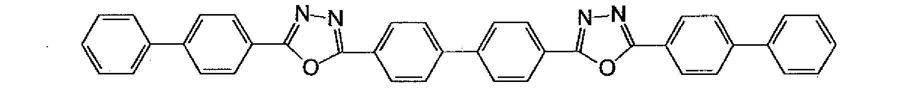

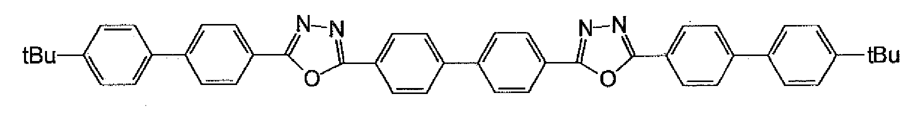

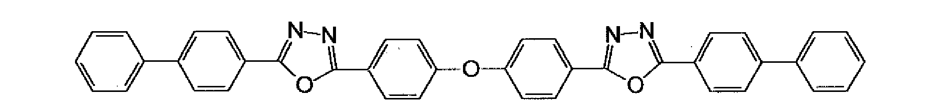

Further, compounds represented by the following formula are also preferred.
- Nitrogen-containing heterocyclic ring derivatives 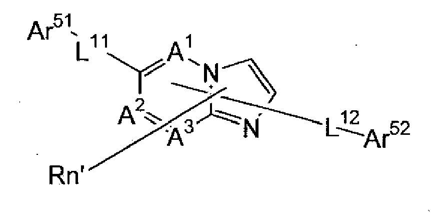 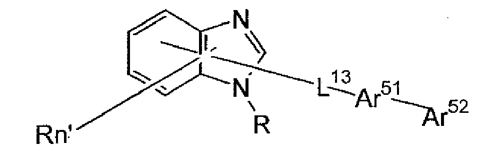 wherein A¹ to A³ are a nitrogen atom or carbon atom; wherein R is a substituted or unsubstituted aryl group having 6 to 60 carbon atoms, a substituted or unsubstituted heteroaryl group having 3 to 60 carbon atoms, an alkyl group having 1 to 20 carbon atoms, a haloalkyl group having 1 to 20 carbon atoms, or an alkoxy group having 1 to 20 carbon atoms, and n' is an integer of 0 to 5, provided that, when n' is an integer of 2 or more, Rs may be the same or different;

adjacent Rs may be bonded to form a substituted or unsubstituted carbocyclic aliphatic ring or a substituted or unsubstituted carbocyclic aromatic ring;
Ar⁵¹ is a substituted or unsubstituted aryl group having 6 to 60 carbon atoms or a substituted or unsubstituted heteroaryl group having 3 to 60 carbon atoms;
Ar⁵² is a hydrogen atom, an alkyl group having 1 to 20 carbon atoms, a haloalkyl group having 1 to 20 carbon atoms, an alkoxy group having 1 to 20 carbon atoms, a substituted or unsubstituted aryl group having 6 to 60 carbon atoms, or a substituted or unsubstituted heteroaryl group having 3 to 60 carbon atoms;
provided that one of Ar⁵¹ and Ar⁵² is a substituted or unsubstituted condensed ring group having 10 to 60 carbon atoms or a substituted or unsubstituted heterocondensed ring group having 3 to 60 carbon atoms;
L¹¹, L¹² and L¹³ are independently a single bond, a substituted or unsubstituted condensed ring having 6 to 60 carbon atoms, a substituted or unsubstituted heterocondensed ring having 3 to 60 carbon atoms or a substituted or unsubstituted fluorenylene group.

• Nitrogen-containing heterocyclic ring derivatives represented by the following formula:

HAr-L^{1'}-Ar^{1'}-Ar^{2'}

wherein HAr is a substituted or unsubstituted nitrogen-containing heterocycle having 3 to 40 carbon atoms,
L^{1'} is a single bond, a substituted or unsubstituted arylene group having 6 to 60 carbon atoms, a substituted or unsubstituted heteroarylene group having 3 to 60 carbon atoms or a substituted or unsubstituted fluorenylene group.
Ar^{1'} is a substituted or unsubstituted divalent aromatic hydrocarbon group having 6 to 60 carbon atoms,
Ar^{2'} is a substituted or unsubstituted aryl group having 6 to 60 carbon atoms or a substituted or unsubstituted heteroaryl group having 3 to 60 carbon atoms.

• Silacyclopentadiene derivatives represented by the following formula: 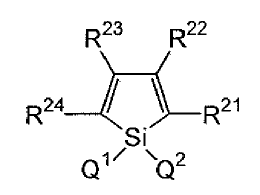 wherein Q¹ and Q² are independently a saturated or unsaturated hydrocarbon group having 1 to 6 carbon atoms, an alkoxy group, an alkenyloxy group, an alkynyloxy group, a hydroxyl group, a substituted or unsubstituted aryl group, or a substituted or unsubstituted hetero ring, or Q¹ and Q² are bonded to form a saturated or unsaturated ring, and R²¹ to R²⁴ are independently hydrogen, halogen, a substituted or unsubstituted alkyl group having 1 to 6 carbon atoms, an alkoxy group, an aryloxy group, a perfluoroalkyl group, a perfluoroalkoxy group, an amino group, an alkylcarbonyl group, an arylcarbonyl group, an alkoxycarbonyl group, an aryloxycarbonyl group, an azo group, an alkylcarbonyloxy group, an arylcarbonyloxy group, an alkoxycarbonyloxy group, an aryloxycarbonyloxy group, a sulfinyl group, a sulfonyl group, a sulfanyl group, a silyl group, a carbamoyl group, an aryl group, a heterocyclic group, an alkenyl group, an alkynyl group, a nitro group, a formyl group, a nitroso group, a formyloxy group, an isocyano group, a cyanate group, an isocyanate group, a thiocyanate group, an isothiocyanate group, or a cyano group, and adjacent groups of R²¹ to R²⁴ form a substituted or unsubstituted condensed ring.)

• Silacyclopentadiene derivatives represented by the following formula: 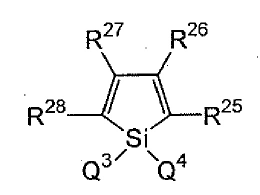 wherein Q³ and Q⁴ are independently a saturated or unsaturated hydrocarbon group having 1 to 6 carbon atoms, alkoxy group, alkenyloxy group, alkynyloxy group, substituted or unsubstituted aryl group, or substituted or unsubstituted hetero ring, or Q³ and Q⁴ are bonded to form a saturated or unsaturated ring, and R²⁵ to R²⁸ are independently hydrogen, halogen, substituted or unsubstituted alkyl group having 1 to 6 carbon atoms, alkoxy group, aryloxy group, perfluoroalkyl group, perfluoroalkoxy group, amino group, alkylcarbonyl group, arylcarbonyl group, alkoxycarbonyl group, aryloxycarbonyl group, azo group, alkylcarbonyloxy group, arylcarbonyloxy group, alkoxycarbonyloxy group, aryloxycarbonyloxy group, sulfinyl group, sulfonyl group, sulfanyl group, silyl group, carbamoyl group, aryl group, heterocyclic group, alkenyl group, alkynyl group, nitro group, formyl group, nitroso group, formyloxy group, isocyano group, cyanate group, isocyanate group, thiocyanate group, isothiocyanate group, or cyano group, or adjacent groups of R²⁵ to R²⁸ form a substituted or unsubstituted condensed ring, (provided that, when R²⁵ and R²⁸ are phenyl groups, Q³ and Q⁴ are neither an alkyl group nor a phenyl group, when R²⁵ and R²⁸ are thienyl groups, a case is excluded in which Q³ and Q⁴ are monovalent hydrocarbon groups and R²⁶ and R²⁷ are an alkyl group, an aryl group, an alkenyl group, or R³⁶ and R³⁷ are aliphatic groups which form a ring by bonding to each other, when R²⁵ and R²⁸ are silyl groups, R²⁶, R²⁷, Q³, and Q⁴ are neither independently a monovalent hydrocarbon group having 1 to 6 carbon atoms nor a hydrogen atom, and when a benzene ring is condensed at the positions of R²⁵ and R²⁶, Q³ and Q⁴ are neither an alkyl group nor a phenyl group) .

• Borane derivatives represented by the following formula: 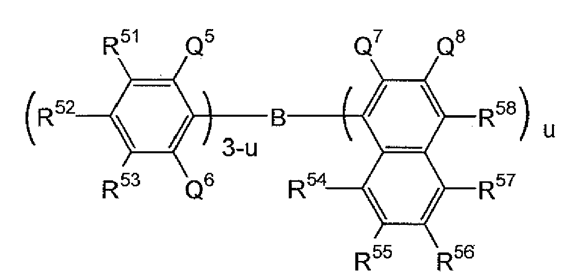 wherein R⁵¹ to R⁵⁸ and Q⁸ are individually a hydrogen atom, a saturated or unsaturated hydrocarbon group, an aromatic group, a heterocyclic group, a substituted amino group, a substituted boryl group, an alkoxy group, or an aryloxy group, Q⁵, Q⁶, and Q⁷ are individually a saturated or unsaturated hydrocarbon group, an aromatic group, a heterocyclic group, a substituted amino group, an alkoxy group, or an aryloxy group, the substituents for Q⁷ and Q⁸ may be bonded to form a condensed ring, u is an integer of 1 to 3, provided that the Q⁷s may differ when u is 2 or more, and a case in which u is 1, Q⁵, Q⁶, and R⁴⁰ are methyl groups, and R⁵⁸ is a hydrogen atom or a substituted boryl group, and a case in which u is 3 and Q⁷ is a methyl group are excluded.

• Compounds repesented by the following fomrula: 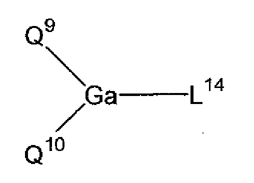 wherein Q⁹ and Q¹⁰ are independently a ligand represented by the following formula; and L¹⁴ is a halogen atom, a substituted or unsubstituted alkyl group, a substituted or unsubstituted cycloalkyl group, a substituted or unsubstituted aryl group, a substituted or unsubstituted heterocyclic group, -OR^{a} wherein R^{a} is a hydrogen atom, a substituted or unsubstituted alkyl group, a substituted or unsubstituted cycloalkyl group, a substituted or unsubstituted aryl group, a substituted or unsubstituted heterocyclic group, or -O-Ga-Q¹¹(Q¹²) wherein Q¹¹ and Q¹² are the same ligands as Q⁹ and Q¹⁰.

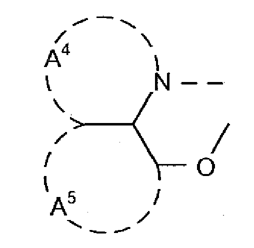

wherein rings A⁴ and A⁵ are each a 6-membered aryl ring structure which may have a substituent, and are condensed to each other.

A preferred embodiment of the invention is a device containing a reducing dopant in an electron-transporting region or in an interfacial region between the cathode and the organic layer. The reducing dopant is defined as a substance which can reduce an electron-transporting compound. Accordingly, various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.

More specific examples of the preferred reducing dopants include at least one alkali metal selected from the group consisting of Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). Metals having a work function of 2.9 eV or less are particularly preferred.
Among these, a more preferable reducing dopant is at least one alkali metal selected from the group consisting of K, Rb and Cs. Even more preferable is Rb or Cs. Most preferable is Cs.

These alkali metals are particularly high in reducing ability. Thus, the addition of a relatively small amount thereof to an electron-injecting zone improves the luminance of the organic EL device and make the lifetime thereof long. As a reducing dopant having a work function of 2.9 eV or less, combinations of two or more alkali metals are preferable, particularly combinations including Cs, such as Cs and Na, Cs and K, Cs and Rb, or Cs, Na and K are preferable.
The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By forming the electron-injecting layer, a current leakage can be effectively prevented and electron-injecting properties can be improved.
As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved.

Specifically preferable alkali metal calcogenides include Li₂O, LiO, Na₂S, Na₂Se and NaO and preferable alkaline earth metal calcogenides include CaO, BaO, SrO, BeO, BaS and CaSe. Preferable halides of alkali metals include LiF, NaF, KF, LiCl, KCl and NaCl. Preferable halides of alkaline earth metals include fluorides such as CaF₂, BaF₂, SrF₂, MgF₂ and BeF₂ and halides other than fluorides.

Semiconductors forming an electron-transporting layer include one or combinations of two or more of oxides, nitrides, and oxidized nitrides containing at least one element of Ba, Ca, Sr, Yb, Al, Ga, In, Li, Na, Cd, Mg, Si, Ta, Sb and Zn. An inorganic compound forming an electron-transporting layer is preferably a microcrystalline or amorphous insulating thin film. When the electron-transporting layer is formed of the insulating thin films, more uniformed thin film is formed whereby pixel defects such as a dark spot are decreased. Examples of such an inorganic compound include the above-mentioned alkali metal calcogenides, alkaline earth metal calcogenides, halides of alkali metals, and halides of alkaline earth metals.

### (Cathode)

For the cathode, the following may be used: an electrode substance made of a metal, an alloy or an electroconductive compound, or a mixture thereof which has a small work function (for example, 4 eV or less). Specific examples of the electrode substance include sodium, sodium-potassium alloy, magnesium, lithium, magnesium/silver alloy, aluminum/aluminum oxide, aluminum/lithium alloy, indium, and rare earth metals.
This cathode can be formed by making the electrode substances into a thin film by vapor deposition, sputtering or some other method.

In the case where light is emitted from the emitting layer through the cathode, the cathode preferably has a light transmittance of larger than 10%.
The sheet resistance of the cathode is preferably several hundred Ω/□ or less, and the film thickness thereof is usually from 10 nm to 1 µm, preferably from 50 to 200 nm.

### (Insulating layer)

In the organic EL device, pixel defects based on leakage or a short circuit are easily generated since an electric field is applied to the ultrathin film. In order to prevent this, it is preferred to insert an insulative thin layer between the pair of electrodes.
Examples of the material used in the insulating layer include aluminum oxide, lithium fluoride, lithium oxide, cesium fluoride, cesium oxide, magnesium oxide, magnesium fluoride, calcium oxide, calcium fluoride, cesium fluoride, cesium carbonate, aluminum nitride, titanium oxide, silicon oxide, germanium oxide, silicon nitride, boron nitride, molybdenum oxide, ruthenium oxide, and vanadium oxide.
A mixture or laminate thereof may be used.

### (Example of fabricating organic EL device)

The organic EL device can be fabricated by forming an anode, an emitting layer, optionally a hole-injecting layer, optionally an electron-injecting layer, and further forming a cathode using the materials and methods exemplified above. The organic EL device can be fabricated in the order reverse to the above, i.e., the order from a cathode to an anode.

An example of the fabrication of the organic EL device will be described below wherein the following layers are successively formed on a transparent substrate:
anode/hole-transporting layer/emitting
layer/electron-transporting layer/cathode.
First, a thin film made of an anode material is formed into a thickness of 1 µm or less, preferably 10 to 200 nm on an appropriate transparent substrate by vapor deposition, sputtering or some other method, thereby forming an anode.
Next, a hole-transporting layer is formed on this anode. As described above, the hole-transporting layer can be formed by vacuum deposition, spin coating, casting, LB technique, or some other method. Vacuum deposition is preferred since a homogenous film is easily obtained and pinholes are not easily generated.

In the case where the hole-transporting layer is formed by vacuum deposition, conditions for the deposition vary depending upon a compound used (a material for the hole-transporting layer), a desired crystal structure or recombining structure of the hole-transporting layer, and others. In general, the conditions are preferably selected from the following: deposition source temperature of 50 to 450°C, vacuum degree of 10⁻⁷ to 10⁻³ torr, vapor deposition rate of 0.01 to 50 nm/second, substrate temperature of -50 to 300°C, and film thickness of 5 nm to 5 µm.

Next, an emitting layer is formed on the hole-transporting layer. The emitting layer can also be formed by making a desired organic luminescent material into a thin film by vacuum vapor deposition, sputtering, spin coating, casting or some other method. Vacuum vapor deposition is preferred since a homogenous film is easily obtained and pinholes are not easily generated. In the case where the emitting layer is formed by vacuum vapor deposition, conditions for the deposition, which vary depending on a compound used, can be generally selected from conditions similar to those for the hole-transporting layer.

Next, an electron-transporting layer is formed on this emitting layer. Like the hole-transporting layer and the emitting layer, the layer is preferably formed by vacuum vapor deposition because a homogenous film is required. Conditions for the deposition can be selected from conditions similar to those for the hole-transporting layer and the emitting layer.

Lastly, a cathode is stacked thereon to obtain an organic EL device.
The cathode is made of a metal, and vapor deposition or sputtering may be used. However, vacuum vapor deposition is preferred in order to protect underlying organic layers from being damaged when the cathode film is formed.
For the organic EL device fabrication that has been described above, it is preferred that the formation from the anode to the cathode is continuously carried out, using only one vacuuming operation.

A method for forming each of the layers constituting the organic EL device of the invention is not particularly limited. A known forming method, such as vacuum deposition, spin coating or the like can be used. The organic thin layer containing the material for the organic EL device of the invention can be formed by a known method, such as vacuum deposition, molecular beam deposition (MBE method), or coating method such as dipping, spin coating, casting, bar coating and roll coating using a solution obtained by dissolving materials in a solvent.

The film thickness of each of the organic layers in the organic EL device of the invention is not particularly limited. In general, defects such as pinholes are easily generated when the film thickness is too small. Conversely, when the film thickness is too large, a high applied voltage becomes necessary, leading to low efficiency. Usually, the film thickness is preferably in the range of several nanometers to one micrometer.

The organic EL device emits light when applying a voltage between electrodes. If a DC voltage is applied to the organic EL device, emission can be observed when the polarities of the anode and the cathode are positive and negative, respectively, and a DC voltage of 5 to 40 V is applied. When a voltage with an opposite polarity is applied, no electric current flows and hence, emission does not occur. If an AC voltage is applied, uniform emission can be observed only when the cathode and the anode have a positive polarity and a negative polarity, respectively. The waveform of the AC applied may be arbitrary.

### EXAMPLES

The material for an organic EL device and the organic EL device of the invention will be described in detail referring to the following examples, which should not be construed as limiting the scope of the invention.

### Producution Example 1

A compound (TH-01) having structural isomers was synthesized by the following process and the process will be described hereinafter.

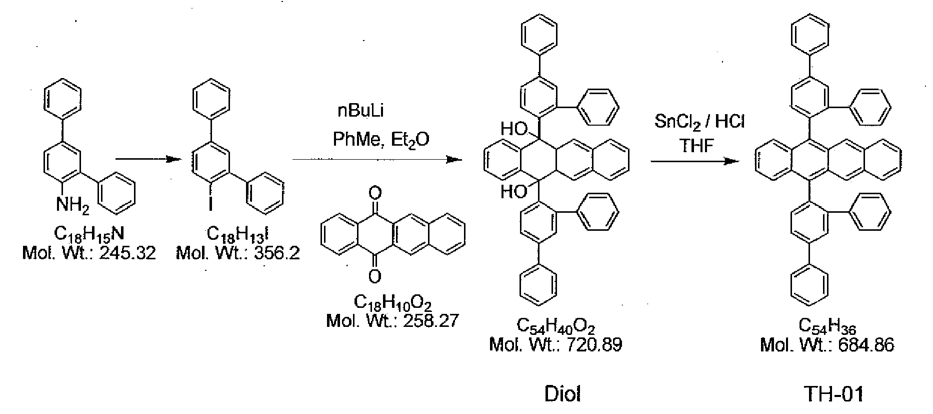

### Synthesis of TH-01

300 mL of 3N aqueous hydrochloric acid was added to 24.5 g of 2,4-diphenylamine in an argon atmosphere. The resulting mixture was heated in an oil bath to 60°C, and then stirred for 4 hours to form a hydrochloride salt (a white suspension).
This white suspension was cooled to 5°C in a salt-ice bath, and then 60 mL of an aquous solution containing 8.27 g of sodium nitrite was dripped for 30 minutes therein while stirring such that the liquid temperature of the white suspension did not exceed 10°C. The reddish brown solution produced was further stirred for 1 hour at 5°C to prepare a diazonium salt solution.
180 mL of an aqueous solution containing 60 g of potassium iodide was prepared in a beaker, and then the diazonium salt solution prepared was gradually added therein for 30 minutes. The resultant solution was further stirred for 30 minutes until generation of nitrogen gas was stopped, and then 200 mL of methylene chloride was added therein to dissolve a product.
After decomposing by-product iodine by adding a small amount of sodium hydrogensulfite, the organic layer was separated and washed with aqueous sodium carbonate and water, followed by drying with magnesium sulfate. The solvent of the resultant organic layer was evaporated under reduced pressure and was purified by column chromatography to obtain 29.4 g of 2,4-diphenyl benzene iodide (yield 82.5%).

27.4 g of 2,4-diphenyl benzene iodide was dissolved in 180 mL of dehydrated toluene and 60 mL of dehydrated ether in an argon atmosphere, and then cooled to -45°C in a dry ice-acetone bath. 31 mL of 2.44 M n-butyl lithium-n-hexane solution was dripped therein for fifteen minutes. The temperature of the resultant solution was gradually raised to -10°C, followed by stirring for 1 hour.
7.75 g of 5,12-naphthacenequinone was gradually added therein for 30 minutes, and the temperature of the resultant mixture was gradually raised to room temperature, followed by further stirring for 5 hours.
The resultant mixture was cooled to 0°C, and 60 mL of methanol was dripped therein. The powder produced was recovered by filtration and was washed with cold methanol several times, followed by vacuum drying to obtain a white powder. 200 mL of toluene was added therein and the resultant mixture was heated and washed for 1 hour, followed by cooling to room temperature. The resultant mixture was filtered, washed with cold toluene and vacuum-dried, thereby obtaining 15.1 g of a white powder of the diol (yield 69.8%).

The following reactions were conducted with a flask equipped with an argon blowing tube, which was shaded with aluminum foil. 450 mL of degassed THF (tetrahydrofuran) was added to 14.42 g of the above-mentioned diol and the resultant mixutre was stirred at room temperature while blowing argon to dissolve the diol. Thereafter, the solution was heated to 40°C inan oil bath. 150 mL of concentrated hydrochloric acid aqueous solution containing 45.1 g of tin(II) chloride dihydrate was dripped therein for 90 minutes. Thereafter, the temperature of the oil bath was raised to 70°C and the resultant mixture was further stirred under reflux for 2 hours, followed by cooling to room temperature.
A 2L beaker was shaded with aluminum foil and 1L of distilled water was added therein and then disgassed by blowing argon flow. The resultant solution was added therein, followed by stirring for 30 minutes. The precipitated yellow powder was recovered by filtration and added in 1L of distilled water again, followed by stirring and washing. The resultant mixture was filtered, sufficiently washed with methanol, and then vacuum-dried. This was heat-washed with 250 mL of aceton disgassed by blowing argon, filtered and vacuum-dried, thereby obtaining 12.70 g of an orange-yellow powder of target TH-01 (yield 92.7%).

For TH-01 and samples A to C mentioned below, the ratio (t/c) of the trans-isomer and the cis-isomer was measured. The measurement was conducted under the follwing conditions by high-performance liquid chromatography (HPLC):
Preparation of sample: A sample was dissolved with THF containing BHT which was disgassed under shading.
Column: Inetrsil ODSIII
Eluent: Acetonitrile/THF (85/25)
Detector: UV 254 nm
The ratio (t/c) was caculated by area ratio (ratio of surface percentages) of each peak in a chart obtained by HPLC measurement.
As a result, TH-01 before heating had a the ratio of the trans-isomer and the cis-isomer of 0.01. Therefore, this TH-01 corresponds to a comparative example of the invention and taken as Comprative example 1.

### Comparative example 2

10.0 g of TH-01 prepared in the Production example was placed into a sublimation refining equipment, and the equipment was filled with nitrogen. The pressure was reduced to 5 x 10⁻⁴ Pa and sublimation refining was conducted at heating temperatures of 320 to 340°C under shading. The treating time was 1 hour. The refined product obtained (8.6 g) was taken as a sample A. The ratio (t/c) of the sample A was 1.0.

### Example 1

6.0 g of the sample A prepared in Comparative example 2 was placed into a sublimation refining equipment again, and the equipment was filled with nitrogen. The pressure was reduced to 5 x 10⁻⁴ Pa and sublimation refining was conducted at heating temperatures of 320 to 340°C under shading. The treating time was 1 hour. The refined product obtained (5.3 g) was taken as a sample B. The ratio (t/c) of the sample B was 2.2.

### Example 2

3.0 g of the sample B prepared in Example 1 was placed into a sublimation refining equipment again, and the equipment was filled with nitrogen. The sample was heated at temperatures of 320 to 340°C for 2 hours under shading and normal pressure. The heated sample was taken as a sample C. The ratio (t/c) of the sample C was 99.
The following organic EL devices were fabricated and evaluated by using TH-01 which had not been heated and the samples A to C which had been subjected to sublimation refining or heating.

### [Organic EL device]

### Example 3

A glass substrate of 25 mm by 75 mm by 1.1 mm thick with an ITO transparent electrode (GEOMATEC CO., LTD.) was subjected to ultrasonic cleaning with isopropyl alcohol for 5 minutes, and cleaned with ultraviolet rays and ozone for 30 minutes.
The resultant substrate with transparent electrode lines was mounted on a substrate holder in a vacuum deposition device. First, a film of the following compound H232 was formed in a thickness of 60 nm so as to cover the surface of the transparence electrode on which the transparence electrode lines were formed. This H232 film functioned as a hole-injecting layer.
The following compound H001 film was further formed in a thickness of 10 nm. This H001 film functioned as a hole-transporting layer.
Thereafter, the sample B prepared in Example 1 as a host material was deposited to form a 40 nm thick film. The following compound RD01 as a dopant material was simultaneously co-deposited at a deposition speed ratio of 1% of the host material. This film functioned as an emitting layer.
The following compound Alq was deposited to form a 30 nm thick film on the above-obtained film. The film served as an electron-injecting layer. Thereafter, LiF was deposited to form a 1 nm thick film as an electron-injecting layer. Metal aluminum was deposited on the Li film to form a metallic cathode, whereby an organic EL emitting device was fabricated.

This organic EL device was measured for a luminous efficiency L/J (cd/A) when the device was driven at 10 mA/cm², and the time priod during which the luminance decreased by 10% (10% decrease in lifetime) when the device was driven at an initial luminance of 5,000 nits at room temperature by a DC constant current. The results are shown in Table 1.
The emission color of each organic EL device was red.

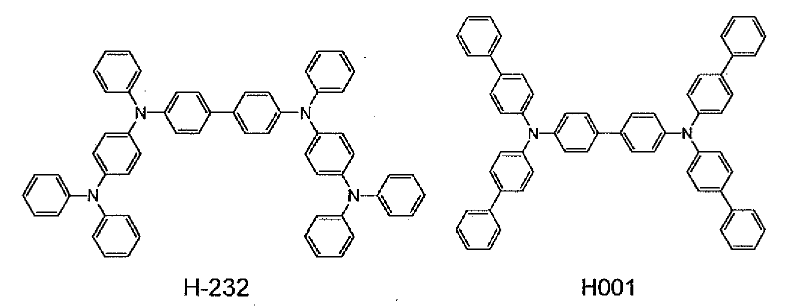

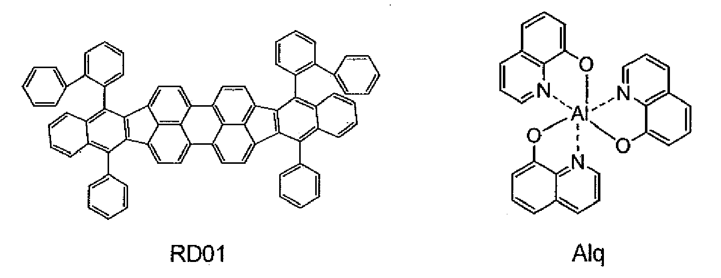

### Example 4, and Comparative examples 3 and 4

An organic EL device was fabricated and evaluated in the same manner as in Example 3, except that a compound shown in Table 1 was used as a host material. Table 1 shows the results.

**Table 1**

| | Host material | t/c of host material | Lunimous efficiency [cd/A] | 10% decrease lifetime [h] |
|---|---|---|---|---|
| Exmaple 3 | Sample B | 2.2 | 6.8 | 200 |
| | (Example 1) | | | |
| Example 4 | Sample C | 99 | 7.2 | 500 |
| | (Example 2) | | | |
| Comparative Example 3 | TH-01 | 0.01 | 4.5 | 50 |
| | (Comparative Example 1) | | | |
| Comparative Example 4 | Sample A | 1.0 | 4.8 | 70 |
| | (Comparative Example 2) | | | |

### INDUSTRIAL APPLICABILITY

The material for an organic EL device of the invention is suitable as a constitution material of an organic EL device, particularly, an emitting layer.
The organic EL device of the invention can be suitably used as a light source such as a planar emitting body and backlight of a display, a display part of a portable phone, PDA, a car navigator, or an instruction panel of an automobile, an illuminator, and the like.

## Claims

1. A material for an organic electroluminescence device which can be a cis-isomer or a trans-isomer of structural isomers,
the content of the trans-isomer being more than that of the cis-isomer.

2. The material for an organic electroluminescence device according to claim 1 wherein the ratio (t/c) of the trans-isomer (t) and the cis-isomer (c) is 2 or more.

3. The material for an organic electroluminescence device according to claim 1 which is represented by the following formula (I): 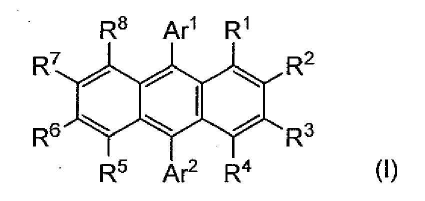 wherein Ar¹ and Ar² are a substituted or unsubstituted aryl group having 6 to 40 ring carbon atoms, or a substituted or unsubstituted heteroaryl group having 5 to 40 ring atoms;
R¹ to R⁸ are a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
R¹ to R⁸ may be the same or different, and adjacent groups thereof may form a saturated or unsaturated ring structure; and
Ar¹ and Ar² have a relation of forming structural isomers.

4. The material for an organic electroluminescence device according to claim 3 wherein Ar¹ and Ar² of the formula (I) are an aryl group represented by the following formula (II): 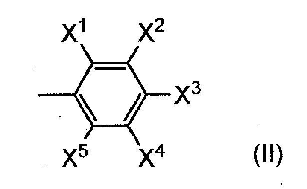 wherein X¹ is a group forming a structural isomer, and is an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group;
X² to X⁵ are each independently a hydrogen atom, an alkyl group having 1 to 20 carbon atoms, an alkoxy group having 1 to 20 carbon atoms, a substituted or unsubstituted aryl group having 6 to 40 ring carbon atoms, a substituted or unsubstituted heteroaryl group having 5 to 40 ring atoms, a halogen atom, a cyano group or a nitro group; and
X¹ to X⁵ may be the same or different, and adjacent groups thereof may form a saturated or unsaturated ring structure.

5. An organic electroluminescence device comprising:
a pair of electrodes, and
one or a plurality of organic layer(s) comprising an organic emitting layer, interposed between the pair of electrodes;
the organic layer(s) containing the material for an organic device of any one of claims 1 to 4.

6. The organic electroluminescence device according to claim 5 wherein the emission zone of the organic layer(s) contain the material for an organic electroluminescence device.

7. The organic electroluminescence device according to claim 5 wherein the emitting layer contains the material for an organic electroluminescence device.

8. The organic electroluminescence device according to claim 7 wherein the emitting layer contains the material for an organic electroluminescence device as a main material.

9. A method for producing a material for an organic electroluminescence device comprising:
heating a mixture of a cis-isomer and trans-isomer of the material which are structural isomers to cause the ratio (t/c) of the trans-isomer (t) and the cis-isomer (c) to be 2 or more.
